Europäisches Patentamt

**European Patent Office**

**Office européen des brevets**

(19)

(11) Publication number: **0 343 306**
**A1**

# EUROPEAN PATENT APPLICATION

(12)

(21) Application number: **88500049.7**

(22) Date of filing: **25.05.88**

(51) Int. Cl.4: **A61K 31/52 , A61K 47/00**

The title of the invention has been amended (Guidelines for Examination in the EPO, A-III, 7.3).

(43) Date of publication of application:
**29.11.89 Bulletin 89/48**

(84) Designated Contracting States:
**AT BE DE FR GB GR IT LU NL SE**

(71) Applicant: **CARULLA VEKAR, S.A.**
**Crta. Naciaonal 1, Km. 16,200**
**Alcobendas-Madrid(ES)**

(72) Inventor: **Gallo Molina, Ramon**
**c/ Moscardo, 18**
**Madrid(ES)**

(74) Representative: **Gonzalez Sanchez, Francisco Javier**
**Po. de la Castellana, 8**
**E-28046 Madrid(ES)**

(54) **Liquid pharmaceutical preparation based on theophylline with a retarding effect, and process for producing it.**

(57) This preparation comprises a hydrocolloid solution of theophylline in a phytocolloid obtained from marine algae, along with excipients and liquid vehicles pharmaceutically acceptable, where the active component remains linked to the colloid by the polar charges of the chains of this latter and at the same time is trapped and dissolved in the liquid phase of the gel, in colloid form.

The procedure for its obtention is initiated with the preparation of an aqueous solution of a phytocolloid. This solution is added to a syrup, based on sweetening, flavouring and colouring agents, dissolution of the active principle in another part of the phytocolloid solution and add slowly this latter solution to the first one with continuous shaking.

EP 0 343 306 A1

## LIQUID PHARMACEUTICAL PREPARATION ON THE BASIS OF THEOPHYLLINE WITH RETARD EFFECT AND PROCEDURE FOR ITS OBTENTION

### DESCRIPTION

OBJECT OF THE INVENTION

The present invention is connected with a pharmaceutical preparation in liquid form, for oral use, in which the pharmacological action of its active principle, THEOPHYLLINE, is modified with a retarded pharmacokinetic activity.

The invention is also connected with a process for the obtention of said pharmaceutical preparation.

BACKGROUND OF THE INVENTION

The possibilities of favourably modifying the characteristics both of absorption and of elimination of an active principle, has constituted a stimulus for pharmaceutical research and for doctors during the last 25 years.

As a general rule it can be stated that a drug is more or less efficacious when its level free of pharmacodynamic substrate, exercising its therapeutic action, is maintained at a determined concentration which is constant in time.

Recent pharmacological research has shown that, with many drugs, the patient responds better to minimal and prolonged doses. On the other hand, recent pharmacodynamic studies, concerning the use of theophylline, have modified knowledge on the use of theophylline in clinical medicine.

The need for the chronic treatment of asthma with theophylline has developed the prolonged action galenic form in recent years.

These preparations contain a sufficient amount of active principle theophylline, and administered at intervals, liberate the theophylline regularly into the blood stream. The plasmatic values of theophylline must be stable.

At present the forms with prolonged action most used are the solid forms (coated tablets, tablets and capsules) with retarded action or with continuous programmed liberation.

In this second form, which is a modificiation of the former, two or three phases of active principle liberation usually play a part: a relatively rapid phase, a semi-slow phase and another slow phase.

The retarded liberation in a pharmaceutical product is that by which, supplied to the patient in sufficient amount to achieve the desired pharmacological effect, provides a sustained activity at therapeutical level in the number of hours desired; for the drugs in which their activity is in proportion with the level in blood, the concept of "pharmacological action" could be substituted by that of "level".

DESCRIPTION OF THE INVENTION

In accordance with the present invention a new galenic form is supplied for the administration of theophylline by oral route, which shares the advantages of the liquid form with regard to its easy handling respecting dosification and with respect to the patients, children and old people, with problems in swallowing solid forms, and the advantages of the retarded action preparations which are at the moment presented in solid forms (tablets, coated tablets and capsules).

The new pharmaceutical composition provided by this invention, has the advantage, in the sphere of theophylline products, to place at the doctor's disposal a liquid theophylline preparation, with a sustained liberation of theophylline. At the same time, and through its galenic characteristics, the preparation according to the invention modifies the bioavailability of theophylline in hydroalcoholic solution, placing it with a bioavailability which is considered sustained, with an average of about 10 hours, without reaching the initial serous peak which characteristic of the theophyllines in hydroalcoholic solution; this latter being an advantage which brings the patients with difficulties in swallowing solid forms.

The invention theophylline hydrocolloid solution, containing in addition, colouring agents, sweeteners

2

and aromatic agents, already traditional in the pharmaceutical sphere, is presented as a more or less viscose liquid, homogeneous at first sight, and stable.

According to the second objective of the invention, the process for obtaining the liquid theophylline preparation, this is characterized by dissolving the active principle, all or part, in a phytocolloid obtained from marine algae. The active principle, theophylline, remains linked to the colloid by the polar charges of the chains of this latter and at the same time is trapped and dissolved in the liquid phase of the gel, in colloid form.

Therefore, the active principle remains, all or part, included in the gel.

The phytocolloid used for the obtention of the theophylline preparation is contained in some marine algae by the process of extraction, gelling compression and desiccation.

The phytocolloid resulting from the extraction of these algae, is formed by two perfectly differentiated fractions: Agarose and Agaropectin Polysaccharides formed by D-galactose and 3,6 anhydrous L-galactose, with different degrees of polymerization.

In order to obtain the theophylline preparation, the Agaropectin content and its gelling power must be normalized in the phytocolloid.

The liquid theophylline preparation with retarded action and the process for its obtention, according to the invention, is illustrated as follows by the following example, which must not be considered as limiting the scope of the invention.

## EXAMPLE

Formula for 100 litres of syrup:

| | | |
|---|---|---|
| Anhydrous Theophylline . . . . . . . . | 533 | grs. |
| Sodium Saccharin . . . . . . . . . . | 20 | grs. |
| Methylparaben . . . . . . . . . . . | 150 | grs. |
| Propylparaben . . . . . . . . . . . | 50 | grs. |
| Saccharose . . . . . . . . . . . . . | 20 | Kgs. |
| Bright red colouring . . . . . . . . | 0.04 | grs. |
| Erythrosine certolake . . . . . . . | 0.02 | grs. |
| Strawberry essence . . . . . . . . . | 15 | c.c. |
| Desiccated phytocolloid . . . . . . | 4 | Kgs. |
| Distilled water . . . . . s.q.f. . | 100 | lts. |

In a first phase (a) a syrup is prepared with the 20 grs. saccharose plus 20 grs. sodium saccharine, 50 grs. propylparaben, 150 grs. methylparaben, 0,02 grs. red colouring and 0,01 grs. erythrosine certolake, up to a volume of 45 litres. The previously prepared syrup is filtered.

In a second phase (b) the 40000 grs. of phytocolloid are dissolved apart at boiling point and continuous shaking. Once the phytocolloid is perfectly dissolved it is kept shaking continuously, lowering the temperature to 60° C.

In a third phase (c), 12 litres of the previously prepared phytocolloid solution (b) are dissolved by shaking in the previously prepared syrup (a).

Once the pH of the phytocolloid (b) is stabilised to pH 6.8 ± o.1 and a temperature of 60° C, the THEOPHYLLINE active principle is added to the solution with continuous shaking, together with the rest of the colourings and once dissolved, the temperature is lowered to 50° C.

Lastly, in the solution (c) formed by the syrup and the phytocolloid and at a temperature of 15° C, with constant shaking, the phytocolloid which contains the THEOPHYLLINE active principle is added, keeping constant the number of shaking revolutions and the temperature of the resulting mixture. Once the active principle colloid has been incorporated it is topped up with water.

Next a study is shown of the pharmacokinetic behaviour of the liquid theophylline formula, according to the present invention, in order to compare the bioavailability of the new theophylline formula according to the invention with a theophylline specialty already on the market (TEOLIXIR) as a reference product and one of known composition.

As trial material a liquid theophylline formula in accord with the invention, with the following composition was used;

| Anhydrous Theophylline | 533 mg. |
|---|---|
| Methylparaben | 150 mg. |
| Propylparaben | 50 mg. |
| Sodium Saccharine | 20 mg. |
| Saccharose | 20 mg. |
| Polyglucide gel s.q.f. | 100 ml. |

In this trial white Wister rats were used, all males, who were 6 months old at the start and with a weight between 350 to 450 grs. fed as usual, with special Panlab rat food.

Each product to be tested was diluted in equal parts with "white" excipient. With the reference product (TEOLIXIR), 20˚ was used as white excipient and with the invention product the polyglucide gel indicated in the previous formula. The dilutions were made with 5 ml. of product and 5 ml. white excipient in 15 ml. test tube and shaken in 20 to 30 shakes.

The administration was made in the rats by gastric sound as is usual, with 1 ml. syringe, and administering 0.60 ml. to 420 grs. rats, normalizing the rats to 420 grs. and increasing or reducing the 0.60 ml. in accordance with the weight differences.

Before the administration, 24 hours before, the rats were not fed and they were slightly anesthetized with ether.

The extraction of blood was made at the times which had passed after the administration. For this, the rats were anesthetized with ether and about 1 ml. blood was extracted from the animals' tails. Half way through the extraction 2 drops of heparin solution were added. This was centrifuged and the plasma obtained put into another small tube.

The results obtained are shown in the following Tables 1 and 2.

**TABLE 1.   Results of plasmatic levels of the invention formula.**

| Experiment N° | Weight of Rat (grs.) | Time passed (hours) | Level 8/ml. |
|---|---|---|---|

## TABLE 1 (Continuation)

| | | | | |
|---|---|---|---|---|
| 1 | 395 | 3 | 7.05 | _ |
| 2 | 420 | 3 | 6.60 | x = 6.93 |
| 3 | 400 | 3 | (5.07) | n = 3 |
| 4 | 415 | 3 | 7.14 | |
| | | | | |
| 5 | 350 | 9 | 4.71 | _ |
| 6 | 360 | 9 | 4.50 | x = 4.266 |
| 7 | 400 | 9 | 4.71 | s = 0.6136 |
| 8 | 380 | 9 | 3.24 | n = 5 |
| 9 | 420 | 9 | 4.17 | c.v. = 14.38 |

## TABLE 2. Plasmatic levels with Teolixir (reference product).

| Experiment N° | Weight of Rat (grs.) | Time passed (hours) | Level 8/ml. | |
|---|---|---|---|---|
| 10 | 370 | $3^{20}$ | 3.22 | |
| 11 | 390 | $3^{20}$ | 4.96 | $\bar{x}$ = 4.95 |
| 12 | 460 | 3 | 4.88 | n = 3 |
| 13 | 430 | 3 | (2.73) | |
| | | | | |
| 14 | 330 | $8^{50}$ | 4.02 | |
| 15 | 360 | 9 | 2.68 | x = 3.37 |
| 16 | 400 | $8^{50}$ | 2.95 | s = 1.43 |
| 17 | 420 | 9 | 3.12 | n = 5 |
| 18 | 380 | 9 | 2.34 | c.v. = 42.43 |
| 19 | 450 | 9 | 1.75 | |

The bioavailability differences between the two formulas tested were shown by means of the determination, at two different times, of the plasmatic levels.

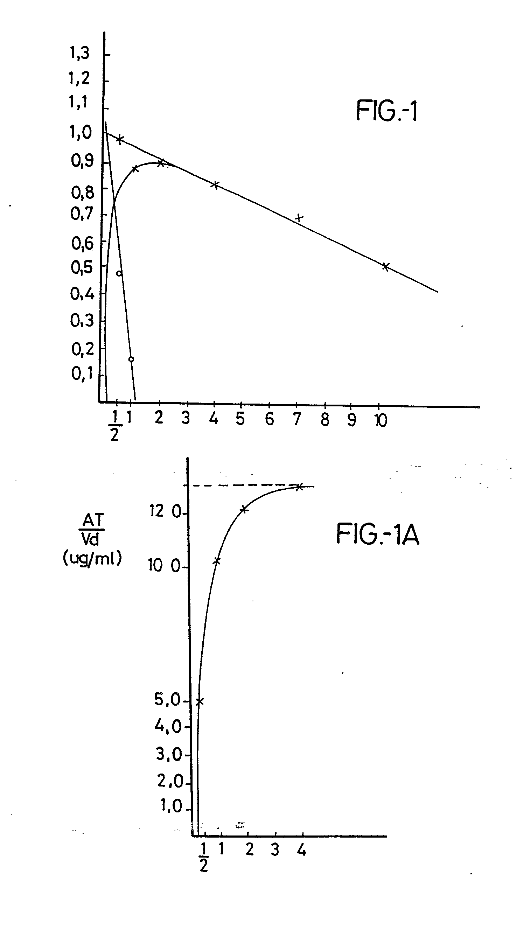

The time of 3 hours was selected, on estimating that this time was at the end of the maximum level zone of the invention formula (see Figure 1) and in the descent zone of Teolixir.

Figure 1 attached illustrates the plasmatic level curve of the invention formula and which was obtained with the administration of 0.6 ml (diluted with white excipient) per rat.

The other reference time selected was that of 9 hours, this due to sensitivity problems, since this analytical technical stopped being reliable for data lower than 1 $\mu$g/ml while at about 3 $\mu$g/ml. a 10% error existed.

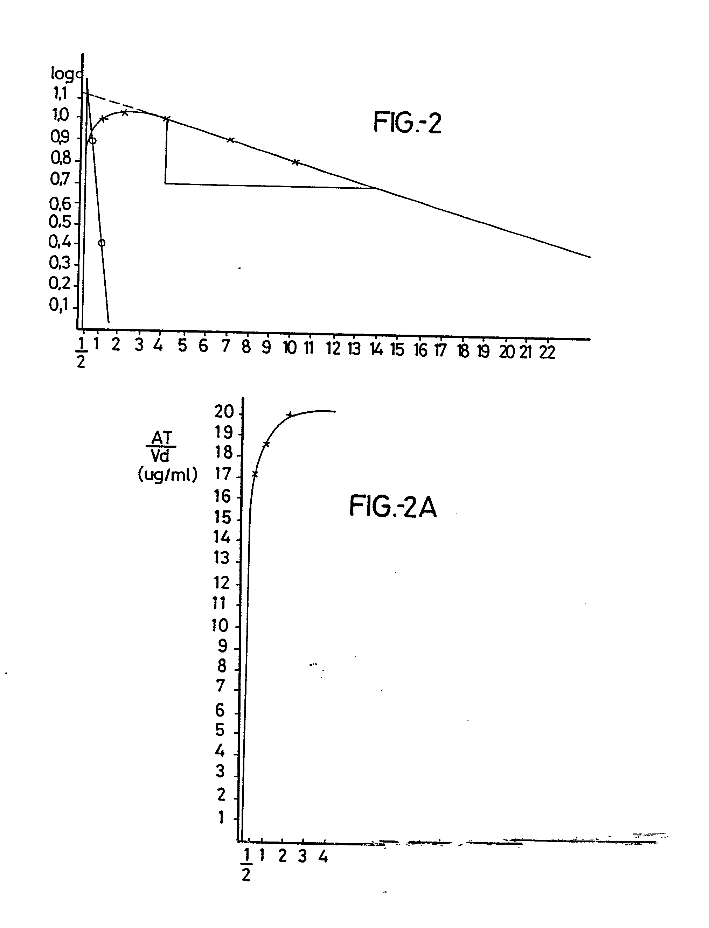

The results of Tables 1 and 2 are shown in the attached Figure 2, from which the following pharmacokinetic parameters are obtained which are shown in Table 3.

**TABLE 3.   Pharmacokinetic Parameters.**

| Parameters | Invention formula | Teolixir |
|---|---|---|
| Half-life (hrs.) | 13.0 | 7.8 |
| Elimination constant ($h^{-1}$) | 0.0533 | 0.0888 |
| Plasmatic level after 3 hrs. (average value of 3 concordant determinations) | 6.91 | 4.97 |

**TABLE 3 (Continuation)**

| | Invention formula | Teolixir |
|---|---|---|
| Plasmatic level after 9 hrs. (average value of N° concordant determinations) | 4.50(4) | 2.77(4) |
| Area between 3 and 9 hours $\dfrac{(y\ 3)}{k_{20}} \quad \dfrac{\mu g}{ml} \times h$ | 45.21 | 18.02 |

From the previous table it can be observed that the bioavailability of the new invention product with reference to Teolixir is:

$$\text{BA (relative)} = \frac{\text{Area of 3 to 9 hours of invention product}}{\text{Area of 3 to 9 hours of Teolixir.}} = \frac{4.52}{1.802} = 2.51$$

Therefore, the invention product is superior in bioavailability to Teolixir.

Another way of determining the bioavailability is by the maxima quotient

$$\text{BA (relative)} = \frac{6.91}{4.50} = 1.54$$

This other expression shows that the bioavailability of Teolixir is surpassed 50% by the invention product.

At the same time, the BA (relative) after 9 hours is

$$\frac{4.50}{2.72} = 7.65$$

which means bioavailability for the product which is about 65% greater.

The half-lives (and elimination constants) show that the invention product is superior to Teolixir at the moment of achieving sustained action.

The results of Teolixir are more disperse, which indicates more erratic pharmacokinetics (V.C. of 14.38 for the invention product and 42.43 for Teolixir).

All the results for the invention product after 9 hours pass the stadistical data exclusion criterion (Chauvenet criterion), the four concordant data being expressed in an average value, the datum to be excluded being enclosed in brackets.

At the same time all the Teolixir values after 9 hours are acceptable with regard to the average value according to the Chauvenet exclusion criterion.

The data of the invention product studied in a $P = 0.1$ it was observed that after 9 hours four concordant data were sufficient, 5 acceptable data and 4 concordant ones having nevertheless been obtained.

As a conclusion, it can be said that the invention product is superior to Teolixir with regard to bioavailability and longer half-life.

Pharmacokinetic studies were also carried out in voluntary persons, according to international norms, the two formulas being compared in a crossed trial, that is to say the one with sustained action and the reference one with unsustained action.

The persons were all males, of an age suitable for these trials, and apparently healthy, with the following parameters shown in

TABLE 4

| Characteristics of the persons. | | | | |
|---|---|---|---|---|
| | A | B | C | D |
| Weight (Kg) | 60 | 70 | 75 | 67 |
| Height (cm) | 180 | 180 | 180 | 168 |
| Age (years) | 23 | 24 | 24 | 24 |

300mg/60kg of weight were administered in fasting conditions to persons who had not eaten any food containing xanthines during the previous 24 hours.

5ml blood were extracted 1/2 hour, 1, 2, 4, 7 and 10 hours after the intake.

The blood with 4 drops of heparin was centrifuged, the plasma removed and analysed.

After two weeks, the trial was repeated with the other preparation (Teolixir).

The results obtained are shown in Tables 5 and 6.

### TABLE 5. Plasmatic levels of the invention product.

| Time (hours) | A (µg/ml) | B (µg/ml) | C (µg/ml) | D (µg/ml) | $\bar{x}$ |
|---|---|---|---|---|---|
| 1/2 | 4.88 | 3.51 | 7.16 | 7.76 | 5.83 |
| 1 | 12.35 | 7.38 | 8.25 | 9.26 | 9.21 |
| 2 | 12.12 | 9.27 | 5.42 | 8.76 | 8.89 |
| 4 | 10.82 | 9.23 | 5.42 | 7.51 | 8.25 |
| 7 | 9.30 | 6.85 | 4.45 | 6.00 | 6.65 |
| 10 | 7.32 | 5.74 | 3.61 | 4.38 | 5.18 |

### TABLE 6

| Plasmatic levels of Teolixir. | | | | | |
|---|---|---|---|---|---|
| Time (hours) | A (µg/ml) | B (µg/ml) | C (µg/ml) | D (µg/ml) | $\bar{x}$ |
| 1/2 | 8.48 | 10.69 | 14.3 | 8.14 | 10.4 |
| 1 | 7.86 | 7.60 | 12.0 | 8.07 | 8.8 |
| 2 | 7.72 | 7.58 | 12.83 | 8.14 | 9.1 |
| 4 | 6.62 | 6.13 | 7.5 | 6.41 | 6.7 |
| 7 | 5.44 | 3.93 | 6.3 | 5.93 | 5.4 |
| 10 | 3.38 | 3.17 | 4.9 | 4.62 | 4.0 |

Figure 1, attached, shows the plasmatic levels of two persons with concordant data regarding Teolixir.

Figure 2, attached, and with the data of the persons shown in averages (A and B), the semi-logarithm representation is shown for the invention product and the pharmacokinetic parameters which are shown in overall form in Table 7 are calculated.

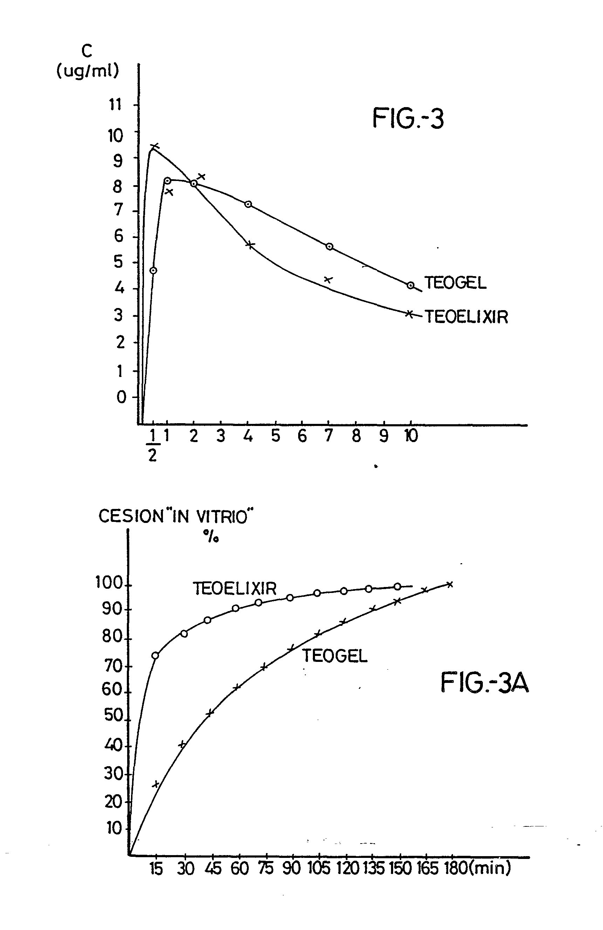

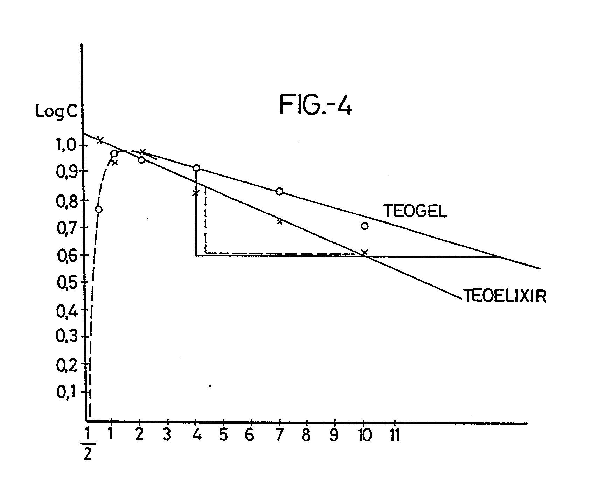

In a similar way, Figure 3, attached, shows the data with Teolixir and the invention product and, in logarithm form, this data is shown in Figure 4.

TABLE 7

| Pharmacokinetic Parameters. | | |
|---|---|---|
| Parameter | Invention Product | Teolixir |
| Elimination half-life | 9.9 h | 6.25 h |
| Elimination constant | 0.07 h$^{-1}$ | 0.111 h$^{-1}$ |
| Absorption half-life | 0.23 h | 0.15 h |
| Absorption constant | 3.01 h$^{-1}$ | 4.62 h$^{-1}$ |
| Latency | <10 min. | <5 min. |
| Acme | 10.96 $\mu$g/ml | 17.70 $\mu$g/ml |
| Maximum | 1 h | 1/2 h |
| Area ($_R^o$) | 192.7 $\mu$g/ml x h | 197.1 $\mu$g/ml x h |
| Bioavailability | 0.978 | 1.0 |

The correlation "vitro-vivo" was studied from the previous experiments.

With the pharmacokinetic data regarding persons the degree of absorption was calculated (Tables 8 and 7) and the "vitro-vivo" correlation (Table 10).

TABLE 8

| Degree of absorption of the invention product (Fig. 1A). | | | | | |
|---|---|---|---|---|---|
| Time (h) | Average plasmatic level $\mu$g/ml | AUC $_{t=0}^{t=T}$ - $\mu$g-h/ml | KeAUC $\mu$g/ml | AT/Vd $\mu$g/ml | % |
| 1/2 | 4.95 | 0.743 | 0.052 | 5.00 | 38.5 |
| 1 | 9.87 | 5.229 | 0.366 | 10.24 | 78.8 |
| 2 | 10.70 | 20.58 | 1.440 | 12.14 | 93.4 |
| 4 | 10.03 | 41.28 | 2.890 | 12.89 | 100.0 |

TABLE 9

| Degree of absorption of Teolixir (Figure 3A). | | | | | |
|---|---|---|---|---|---|
| Time (h) | Average plasmatic level $\mu$g/ml | AUC $_{t=0}^{t=T}$ - $\mu$g-h/ml | KeAUC $\mu$g/ml | AT/Vd $\mu$g/ml | % |
| 1/2 | 16.60 | 4.69 | 0.52 | 17.11 | 85.6 |
| 1 | 17.10 | 13.17 | 1.46 | 18.56 | 92.8 |
| 2 | 16.65 | 30.15 | 3.35 | 20.00 | 100.0 |

The previous degrees of absorption were obtained applying the equation of Pomerana et al.

$$\frac{d\ A}{dt} = Vd \quad \frac{d\ Cb}{d\ t} + KeCb$$

where:

DA/dt    speed of absorption
Vd    apparent distribution volume
d  CB         speed of concentration change in blood

Cb    concentration in blood
Ke    elimination constant

TABLE 10

| Vitro-Vivo Correlation (Figure 3A). | | | | |
|---|---|---|---|---|
| Time (hours) | Teogel | | Teolixir | |
| | Cession in Vitro (%) | Absorption in Vitro (%) | Cession in Vitro (%) | Absorption in Vitro (%) |
| 1/2 | 39.8 | 38.5 | 81.9 | 85.6 |
| 1 | 62.8 | 78.8 | 91.7 | 92.8 |
| 2 | 85.9 | 93.4 | 97.2 | 100.0 |

The theopyhylline derivatives act inhibiting the phosphodiesterase in competitive form. This results from the increase in the intracellular cyclicle AMP and, as a result, from the increase in the liberation of endogenous adrenaline.

The studies made on theophylline pharmacokinetics show that the treatment is efficacious if the plasmatic levels of theophylline are kept in a constant way between 10 and 20 mg/ml. Generally, the plasmatic levels which are lower than 10 mcg/ml do not provide a satisfactory response. Levels above 20 mcg/ml are associated with a significant incidence of side effects. Because of this, the treatment must be aimed at the maintenance of plasmatic levels of theophylline between 10 and 20 mcg/ml.

Due to the enormous individual variations in theophylline elimination, the dose adjustment must be individualised. However, once the optimum dose has been established, the plasmatic levels of theophylline usually remain constant for each patient.

The nature of the invention and the way of making it in practice, having been sufficiently described, it must be stated that the instructions previously indicated are subject to modifications in detail provided they do not alter its basic principle.

## Claims

1. Liquid pharmaceutical preparation on the basis of theophylline with retarded effects, characterised because it comprises an hydrocolloid solution of theophylline in a phytocolloid obtained from marine algae, together with pharmaceutically acceptable excipients and liquid vehicles.

2. Pharmaceutical preparation in accordance with claim 1, characterised because the active component, theophylline, is linked to the colloid by means of the polar charges of the chains of this latter, remaining trapped and dissolved in the liquid phase of the gel in colloid form.

3. Pharmaceutical preparation in accordance with claim 2, characterised because the active principle, theophylline, is included totally or partially in the gel.

4. Pharmaceutical preparation in accordance with the claims 1 -3, characterised because the phytocolloid is derived from marine algae.

5. Pharmaceutical preparation according to the claims 1 - 4, characterised because the phytocolloid is made up by two fractions of agarose and agaropectin polysaccharides formed by D-galactose and 3.6-anhydrous-L-galactose with different grades of polymerization.

6. Processes for the obtaining of a liquid pharmaceutical theophylline preparation with retarded effects, characterised because it comprises the stages of:

a) the preparation of an aqueous phytocolloid solution derived from marine algae, by dissolution of this in water at boiling point;

b) the addition of one part of the phytocolloid solution with shaking and at a temperature of 40 to 60°C approximately, is made to a syrup made up of sweeteners, aromatic and colouring agents, and pharmaceutically acceptable excipients and vehicles.

c) the dissolution in another part of the phytocolloid solution, after the stabilising of its pH to values close to neutrality, and at a temperature close to 40-60°C, of the active theophylline principle; and

d) the slow addition of the phytocolloid solution and active theophylline principle resulting from stage (c) to the solution formed by the syrup and phytocolloid of stage (b), with continuous shaking and at room temperature, so that the active theophylline is linked to the phytocolloid by the polar charges of the chains of this latter, remaining trapped and dissolved at the same time in the liquid phase of the gel, in colloid form.

7. Process according to the claim 6, characterised because the phytocolloid is derived from marine algae.

8. Process according to the claims 6 and 7, characterised because the phytocolloid is constituted by two fractions of agarose and agaropectin polysaccharides formed by D-galatose and 3,6anhydrous-L-galactose with different degrees of polymerization.

FIG.-1

$\frac{AT}{Vd}$
(ug/ml)

FIG.-1A

EP 0 343 306 A1

FIG.-2

FIG.-2A

FIG.-3

FIG.-3A

FIG.-4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | ES-A- 547 013 (CARULLA VEKAR S.A.) <br> * Whole document * <br> ----- | 1-8 | A 61 K 31/52 <br> A 61 K 47/00 |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 23-01-1989 | TZSCHOPPE,D.A. |

EPO FORM 1503 03.82 (P0401)